# EUROPEAN PATENT SPECIFICATION

(11) **EP 4 604 887 B1**
(45) Date of publication and mention of the grant of the patent: **30.09.2026**
(21) Application number: 23793806.3
(22) Date of filing: 20.10.2023
(51) Int. Cl.: A61F 11/00, A61H 39/04

(54) **PRESSURE APPLYING DEVICE**
DRUCKANWENDUNGSVORRICHTUNG
DISPOSITIF D'APPLICATION DE PRESSION

(30) Priority: 21.10.2022 EP 22202922
(43) Date of publication of application: 27.08.2025
(73) Proprietor: Grübl, Klaus, 5280 Braunau/Inn (AT)
(72) Inventor: Grübl, Klaus, 5280 Braunau/Inn (AT)
(74) Representative: Gassner, Birgitta
(86) International application number: PCT/EP2023/079241
(87) International publication number: WO 2024/084026

(56) References cited:
- WO-A1-2019/158674
- WO-A1-2021/250104
- JP-A- 2020 039 663
- JP-B1- 7 018 231
- US-A1- 2015 018 870
- UNBEKANNTER VERFASSER: "3D Scanning Printing In Ear Impressions IEM using Fusion 360", 7 December 2019 (2019-12-07), XP055872253, Retrieved from the Internet <URL:https://hacklab.design/bruxism-tinnitus/2019/12/7/3d-scanning-printing-in-ear-impressions-iem-fusion-360> [retrieved on 20211213]

## Description

### Field of the Invention

The present invention relates to the field of improving health conditions in an individual by an acupressure device. Particularly the invention relates to a method for producing an individual device for improving health conditions in a subject, and to an individual device produced by said method as well.

### Background Art

Bioelectronic medicine progressively comes into focus as a non-pharmaceutical treatment option for various diseases. Specifically, electrical stimulation of the auricular vagus nerve is an emerging technology in the field of bioelectronic medicine with applications in therapy. A large number of physiological processes and bodily states are associated with information transfer between the brain and body. These include diseases mitigating effects and sustainable therapeutic applications ranging from chronic pain diseases, neurodegenerative and metabolic ailments to inflammatory and cardiovascular diseases.

Tinnitus is a phantom sound perception in the ears or head and can arise from many different medical disorders. The condition is very common with a prevalence of 10 to 15%. There is little evidence for effective tinnitus treatments until the recent years. WO2019158674 and WO2021250104 for the first time disclose a device with proved effect for successfully treating tinnitus in afflicted individuals. The device exerts soft pressure at various points at the ear. The device is configured as a bracket with a silicone plug to be located at the region of the posterior auricular muscle. The bracket is made from metal and as such quite stiff and consequently not suited for bearing it during sleep.

3D scanning of the ear and printing ear impressions is commonly used to make in-ear monitors which are specifically used by musicians, people with hearing impairments and people that have tinnitus whom need slim ear plugs to sleep.

US2013/0301845A1 discloses an ear plug system comprising an ear plug device and an electronic control unit. The shape of the ear plug device is selected to comfortably fit into most adults' ear to provide a good seal against external noises.

In order to improve the treatment of tinnitus and/or health conditions in a subject, it would be desirable to have a flexible device at hand that may also be worn during sleep.

Therefore, there is still the need for flexible device for treating tinnitus and/or improving health conditions in an individual wherein the devise is specifically suited to be used during sleep.

### Summary of invention

It is the object of the present invention to provide a flexible device for treating tinnitus and/or improve health conditions in a subject. The object is solved by the subject matter of the present invention.

According to the invention, there is provided a flexible and soft device 10 which fits accurate to the individual subject's external ear.

One embodiment of the invention relates to a method for producing an individual device 10 for improving health conditions in a subject, comprising:
scanning the geometry of the outer ears,
producing a 3D-model of the individual device 10, and
manufacturing the device 10 specifically adapted to the individual geometry of the outer ears of the subject.

Specifically, the method for producing an individual device 10 for improving health conditions in a subject, comprises:
scanning the geometry of the outer ears;
producing a 3D-model of the individual device 10; and
manufacturing the device 10;
wherein the device 10 is specifically adapted
to the individual geometry of the outer ears of the subject;
to apply pressure to the external ear in a way that the position of the pinna is changes;
to exert pressure on acupressure points around to the external ear; and wherein the device 10 is flexible.

Alternatively, a molding material is applied behind the pinna by means of an application device in order to mold the area between the pinna and the skull.

Suitable molding materials are for example selected from the group consisting of monophase impression materials, biphasic impression materials, double-mixed materials or alginates.

Further provided is a kit comprising the molding material, an application device and optionally a processing device punch device in order to bring the impression material into shape after curing.

The kit may further contain fixing means in order to secure the plastic impression behind the ear when worn. This is advantageous since the device is intended to be worn in particular while sleeping, thus preventing it from slipping while sleeping.

A further aspect of the invention relates to a method for producing an individual device for treating a tinnitus condition and/or improving other health conditions, comprising the steps:
(a) applying an impression material to the area behind the pinna and the skull,
(b) allowing the impression material to cure,
(c) removing the cured material; and optional
(d) process the surface of the impression of the ear geometry.

In order to obtain a smooth surface of the impression, it may be necessary to manually process the surface of the impression by milling, polishing, smoothing, etc. Specifically, the surface of the impression of the ear geometry is smoothened after curing. This surface treatment can be carried out, for example, by means of a processing device suitable for this purpose, such as a stamping device.

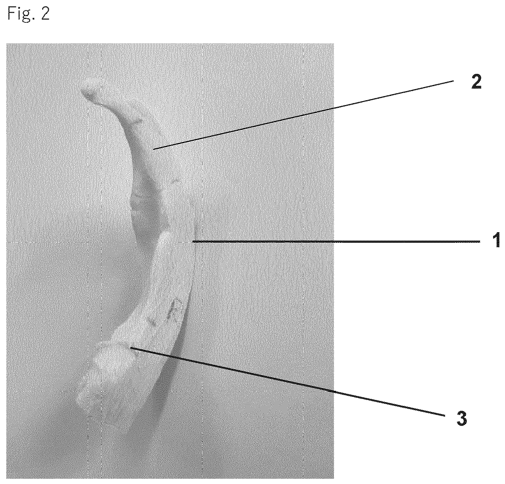

A further embodiment relates to the method as described herein, wherein a silicone impression material is applied to the area between the pinna and the skull with a manual impression gun. According to one embodiment of the invention, a soft vinyl polysiloxane material may be chosen for its low viscosity. When applied, the soft malleable material entered area and cured to the shape of the area between the pinna and head. A fingernail may be used to gently touch the mold to make sure it was set before removing it from the area. A typical set of molds from one subject is depicted in Fig. 2.

The invention relates to the method as described herein, wherein the device 10 comprises an approximately straight portion 20 having circular portion 21, and a curved portion 30, and wherein the device is configured to surround the outer ear.

A further embodiment relates to the method as described herein, wherein the curved portion 30 has a smaller diameter at the beginnings 40, 50 and thickens towards the middle.

A further embodiment relates to the method as described herein, wherein the device 10 is manufactured as one piece.

Specifically, the device 10 comprises a straight portion 20 which is adapted to rest in front of the ear, a curved portion 30 which is adapted to rest behind the ear, and a circular portion 21 which is located in the middle of the straight portion 20.

The invention relates to an individual device 10 produced by a method as described herein, wherein the device fits accurate to the individual subject's outer ear.

Specifically, the individual device 10 produced by a method as described herein relates to the device 10 which fits accurate to the individual subject's outer ear; and wherein the device 10 is specifically adapted
to the individual geometry of the outer ears of the subject;
to apply pressure to the external ear in a way that the position of the pinna is changes;
to exert pressure on acupressure points around to the external ear; and wherein the device 10 is flexible.

The invention relates to the individual device 10 as described herein, wherein the approximately straight portion 20 of the device rest in front of the ear and the curved portion 30 rest behind the ear.

A further example relates to the method as described herein, wherein the curved portion 30 stimulates various acupressure points behind the ear.

A further example relates to the method as described herein, wherein the circular portion 21 stimulates acupressure points in the front ear.

A further example relates to the method as described herein, wherein the curved portion 30 exerts stimuli to the auricular vagus nerve.

One example relates to the use of the individual device 10 as described herein for improving health conditions in a subject.

A further example relates to the use as described herein, wherein the curved portion 30 of the device changes the position of the pinna and stimulates various auricular acupressure points.

A further embodiment relates to the device 10 as described herein, for use in the treatment of tinnitus.

A further example relates to the use of the individual device 10 as described herein, wherein the stimulation of the vagus nerve affect multiple physiological functions.

A further example relates to the use of the individual device 10 as described herein, wherein the multiple physiological functions are selected from the group consisting of neurological disorder, psychometric functions, metabolic syndrome, cardiovascular disorders, and pain.

### Brief description of drawings

Fig. 1 depicts a device 10 comprising an approximately straight portion 20 and a curved portion 30. The straight portion 20 of the device 10 comprises an approximately circular portion 21 which is located in the middle of the straight portion.
Fig. 2 depicts a device 1 comprising an upper 2 and lower part 3 to be worn behind the ear.

### Description of Embodiments

The present invention provides a flexible device to be specifically worn during sleep in order to treat tinnitus and/or to improve health condition of an individual..

The device is according to invention the flexible and soft device is made to surround the outer ear. The device comprises an approximately straight portion and a curved portion. The straight portion of the device comprises a circular portion which is located in the middle of the straight portion. The straight portion may be uniform in thickness.

The curved portion of the device has at the beginnings, i.e., at the upper and lower end, the same diameter as the straight portion for connecting the curved portion and the straight portion. The curved portion thickens towards the middle section.

The external ear, the pinna is the visible part of the ear and also known as the auricle. The function of the external ear is to collect sound by acting as a funnel, amplifying the sound and directing it to the auditory canal The entire outer ear is the pinna. It is divided into different parts. Each individual's pinna creates a distinctive imprint on the acoustic wave traveling into the auditory canal. The numerous elevations and depressions of the auricle form acoustic resonators, which are excited each time sound hits from a certain direction. This creates direction-dependent minima and maxima in the frequency spectrum of the ear signal, which are used by the ear to determine the directions of incidence such as top, bottom, front or rear directional bands.

The middle ear includes the eardrum and the ossicles hammer, anvil and stirrup. The round window connects the scala tympani of the inner ear with the middle ear. The Eustachian tube, also called the ear trumpet, connects the middle ear and nasopharynx. A mechanical impedance conversion takes place in the middle ear, which enables an optimal transmission of the signal from the outer ear to the inner ear. Since the acoustic impedance of water is approximately 3000 times that of air, without the lever system formed by the ossicles, only a small part of the sound energy that reaches the eardrum would be passed on to the inner ear.

The inner ear is located in a small cavity system bony labyrinth within the petrous bone, a portion of the temporal bone. In said bony labyrinth the membranous labyrinth is located, consisting of the cochlea, in which sound is converted into nerve impulses, and the organ of equilibrium. The organ of equilibrium consists of the semicircular ducts and two vesicular portions, the utriculus and the sacculus. The organ of equilibrium is used to detect changes in movement and the direction of gravity. The cochlea and the organ of equilibrium are of similar construction: both are filled with two common parallel fluid systems perilymph and endolymph and have hair cells. The hair cells are cylindrical and are named after the approximately 30 to 150 hair-like extensions at the upper end of the cell stereocilia. The hairs are bent by movements of the fluid and thereby trigger nerve impulses. At the lower end there is a synapse with a sensory neuron. Said synapse releases neurotransmitters even in the resting state. If the hair extensions are deflected by sound vibrations or changes in the movement of the head, the amount of neurotransmitters changes. In the organ of equilibrium, the hair extensions are covered with a kind of gelatinous layer, on which small crystals of calcium carbonate are deposited, which intensify the effect of movements. From the cochlea, the auditory nerve together with the nerve bundles of the organ of equilibrium extend towards the brain as the vestibulocochlear nerve.

The effects of tinnitus strongly depend on the subjective perception and assessment of the ringing in the ears. Ringing in the ears can occur on one or both sides.

Tinnitus aurium means "ringing in the ears". Medically, tinnitus is defined as an acoustic perception that arises from outside the body without a corresponding acoustic stimulus and has no information content.

A distinction is made in principle between two forms. In the case of objective tinnitus, there is a body's own sound source in the ear or near the ear, whose sound emissions are perceived. This means that the sounds that often emanate from the blood vessels or the muscles actually exist and can therefore also be heard by others, even if mostly only with a stethoscope or other medical devices.

Much more common, however, is subjective tinnitus. Here, those affected perceive sounds and noises that cannot be attributed to a physical sound source and therefore cannot be heard by other people. But this does not mean that the patients only imagine the humming, buzzing, whistling, ringing, rustling or knocking. Rather, subjective tinnitus is due to incorrect information formation or processing in the auditory system, which extends from the ear via the auditory nerve to the hearing centers in the brain.

For many of those affected, however, it is not possible to determine definitely the cause of the ringing in the ears. This is called idiopathic tinnitus.

Surprisingly, it has now been found that by changing the position of the outer ear relative to the rest of the ear, the sound is changed, that is to say, the sound is refracted compared to the "normal entry". This leads to a different point of impact on the eardrum. As a result, the hammer handle of the first auditory bone is moved differently and sends changed pressure signals to the next auditory bones or then on to the cochlea. In the cochlea, the sensory hairs stored in a fluid are set in motion differently. This leads to a changed conversion of the electrical signals into the brain and to a change in the learning technology of the synapses - the previous sounds are no longer heard and thus "forgotten" in the long term.

There are many causes of tinnitus, from medications and pain relievers to neck or jaw issues, sleep deprivation and stress, anxiety, and depression. Studies have revealed that tinnitus may be associated with a synchronized hyperactivity in the auditory cortex. If someone has a lot of neck tension or jaw issues, these neurons run alongside the auditory nerve. When they are overactive or on fire, that energy bleeds over to the auditory nerve triggering those neurons to fire. Resulting in the perception of sound in the auditory cortex. Approximately two-thirds of people with tinnitus are able to alter the loudness and pitch of their tinnitus via somatic maneuvers, such as jaw clenching or tensing their neck muscles.

In addition to the change of the position of the outer ear relative to the rest of the ear, the device further addresses multiple acupressure points around the ear. The particularly device is configured to stimulate the muscles behind the ear such as the musculus auricularis superior and the musculus auricularis posterior.

The superior auricular muscle is a muscle above the auricle of the outer ear. It originates from the epicranial aponeurosis, and inserts into the upper part of the medial surface of the auricle. It draws the auricle upwards. The musculus auricularis superior may be stimulated by the upper part 40 of the device which is positioned on the upper part of the ear.

The posterior auricular muscle is a muscle behind the auricle of the outer ear. It arises from the mastoid part of the temporal bone, and inserts into the lower part of the cranial surface of the auricle of the outer ear. It draws the auricle backwards, usually a very slight effect. The musculus auricularis posterior may be stimulated by a portion 30b of the curved part 30 of the device which is positioned on the behind the auricle.

In addition, there is a complex bidirectional interaction between tinnitus and stress. Tinnitus patients often perceive their tinnitus as stressful, intrusive and annoying and a considerable subgroup develops insomnia, attentional and psychological problems such as anxiety or depression as a consequence of the ongoing tinnitus perception. A bidirectional interaction is suggested because stress can also aggravate tinnitus perception. Many patients report that the onset of their tinnitus was preceded by stressful events. The stress level and the affective state mediate the relationship between the loudness of the tinnitus and the individually perceived distress of tinnitus. Furthermore, dynamics of emotions are associated with the course of tinnitus over time, which adds to the complex interaction of tinnitus distress, tinnitus loudness, stress, and emotional perception. This complex interaction was also observed during the first wave of the COVID-19 pandemic.

The curved portion 30 of the device may be of different thickness, depending on the condition to be treated. Same sections 30a,b of the curved portion 30 of the device may be of individually customized thickness in order to address the vagus nerve. The external ear is an ideal place for a non-invasive stimulation of the vagus nerve. In fact, the auricular branch of the vagus nerve surfaces as the auricular afferent vagus nerve and thus forms a cutaneous receptive field in the pinna of the ear. This receptive field may be stimulated by the device according to the invention.

Due to changes in the entry angle of sound, the ear noises tinnitus perceived as "bothering" are no longer perceived, since the sound impinges on the eardrum at other points of impact.

Sound changes frequency due to a different refraction change in path length. Frequency change due to change in distance between observer eardrum and sound source Doppler effect. The sound path in the ear between the outer ear and the eardrum changes. For that reason, the previous "old" frequencies, which were perceived as bothering, are no longer perceived, since they changed in terms of "frequency" and are no longer perceived in the brain. This will mainly occur at high frequencies.

Using the device according to the invention, the entry angle of sound is changed so that those affected no longer hear the previously learned bothering sounds. The device according to the invention therefore comprises a thicker part in the curved portion which is dimensioned to positioned behind the auricle. Thus, due to that thicker part of the device, the position of the auricle is changed. By changing the position of the outer ear, the entry angle of the sound into the ear is changed.

In particular, it is advantageous to apply pressure to the external ear by the device. This pressure can result in that the position of the external ear is changed so that the entry angle of the sound is also changed and thus bothering tinnitus sounds are no longer heard. The perception of noises that are not caused by acoustic signals from the environment is reduced or eliminated altogether with the device according to the invention.

The final appearance of the device 10 is dependent on the individual subject in need of such a device and of the intended function of the device. For the treatment of tinnitus, the thickness of the middle section of the curved portion of the device may be elevated in order to exert a soft pressure at various points behind the ear and to change the position of the pinna. For improving other health conditions, the thickness of the middle section of the curved portion of the device may be elevated to a minor extend in order to exert only a soft pressure at various points behind the auricle without remarkably changing the position of the pinna.

Treatment of tinnitus or other health condition during sleep requires a customized device that can be easily worn during this time without interfering with sleep.

The ear is the closest organ to the brain and the most sensitive one due to its rapid transmission of stimuli. Treatment of various disorders via the ears has been known since centuries. For example, acupressure on certain points of the auricles can relieve headaches, toothaches, backaches, insomnia and other ailments.

Ear acupressure therapy has been used successfully in patients with insomnia (Cha N.H. et al., Holistic Nursing Practice 2017, 102-109). Patients suffering from tinnitus are often affected by insomnia as well. The curved portion of the individual device rests behind the ear and may stimulate respective points. Because of this stimulation, an improvement in sleep could be observed in the wearers of the device according to the invention.

Stress can be defined as any type of change that causes physical, emotional or psychological strain. Stress is your body's response to anything that requires attention or action. Everyone experiences stress to some degree. The way you respond to stress, however, make a big difference to your overall well-being. Stress affects both the brain and body. Little bit of stress is good for people to perform and protect themselves but too much stress can overwhelm them leading to fight, flight or freeze response. So learning how to cope with stress is important for our mental and physical wellbeing.

Stress is a feeling of emotional or physical tension. It can come from any event or thought that makes you feel frustrated, angry, or nervous. Stress affects the psyche in the same way as it affects the state of the body. It can lead to minor and serious illnesses, such as forgetfulness and concentration problems, sleep disorders, limited performance and creativity, nervous restless behavior, and much more.

The vegetative nervous system consists of two components which are active at the same time. One part, the so-called sympathetic nervous system, provides tension, the other part, the parasympathetic nervous system, provides relaxation. Stress leads to tension, and if tension persists, the vegetative nervous system tilts into a mode of overactivation of the sympathetic nervous system, while the parasympathetic nervous system is responsible for reducing stress in the human body.

The curved portion of the individual device rests behind the ear and may stimulate respective points of the vegetative nervous system, more precisely the parasympathetic nervous system. Because of this stimulation, a reduction in stress could be observed in the wearers of the device according to the invention.

Furthermore, studies have indicated a higher prevalence of sexual problems in patients with tinnitus, in particular an association between tinnitus and erectile dysfunction ED has been established. This connection was empirically examined in a Taiwanese study (Cheng Y-F. et al., Sci Rep. 2021, 111, 6982). Consequently, successful treatment of a tinnitus disorder may lead to positive impacts on an erectile dysfunction.

For improving other health conditions, the device further comprises a more or less circular portion 21 at the approximately straight portion 20. This more or less circular portion 21 exerts soft pressure on the tragus.

The vagus nerve is the 10th cranial nerve that starts at the brainstem with two bilateral branches and widely meanders and loops within the neck, thorax, and abdomen. The vagus nerve establishes a mutual connection between the brain and major body structures as pharynx, larynx, trachea, heart, aorta, lungs, and the entire gastrointestinal tract including esophagus, stomach, liver, pancreas, and spleen. The activity of the vagus nerve is proportionally associated with health, wellbeing, relaxation, and even emotions like empathy. The vagus nerve thus plays a crucial role in determining brain-body interactions Kaniusas E., et al. (Front. Neurosci 2019, 13, 1-23).

The external ear is the only place on the body where the vagus nerve sends its only peripheral branch. The auricular branch of vagus nerve surfaces as the afferent auricular vagus nerve and thus forms a cutaneous receptive field in the pinna of the ear. This field is susceptible to external stimuli in terms of peripheral nerve stimulation. In particular, auricular vagus nerve allows for an easy external access via electrical stimulation which then connects directly and favorably the applied stimuli to the brainstem Kaniusas, 2019.

Thus, auricular vagus nerve stimulation is a peripheral, non-pharmacological and minimally invasive neuromodulation technique, altering signal processing in the central nervous system, activating reflex circuitries, exploiting brain plasticity for different therapeutic purposes, and thus, affecting profoundly different areas of the brain Kaniusas, 2019.

Reported clinical application of the auricular vagus nerve stimulations in humans relate to neurological disorders, psychometric functions, metabolic syndrome, cardiovascular disorders, and pain. A positive influence of stimulation of auricular vagus nerve is also foreseen for epilepsy, depression, stroke, Alzheimer's disease, and many other disorders.

Up to date, the auricular vagus nerve is primarily electrically stimulated. Surprisingly, it was found that the device according to the invention also stimulates the auricular vagus nerve through the almost circular part of the approximately straight portion. Upon wearing the device, the circular portion is located at the concha, thus stimulating the auricular vagus nerve.

The device according to the invention is specifically suited for the stimulation of the auricular vagus nerve. According to one embodiment of the invention, the device is configured to be worn while sleeping. This is particularly advantageous because the individual subject must not waste time for attending respective premises where the bioelectronic medicine is applied via electrical stimulation. The device is intended for treating tinnitus and/or stimulating the auricular vagus nerve while sleeping. However, if required, the device may also be worn when awake.

The device may be made as one piece or consist of several pieces which are connected to form one piece. In case the device if produced as one-piece it must be of a certain flexibility in order to be placed around the outer ear. For some embodiments, it may be advantage to provide a device with two or more pieces which are connected to one piece when placed around the outer ear. This will allow in same cases a convenient way to place the device around the outer ear. The two or more pieces are connected in order to gain one piece and to ensure the right fit.

The two or more parts may be connected via a plug-in connector, a connection assembly or via a hook-and-loop-fastener.

The device may be made of synthetic material, e.g., of a plastic material. Preferably, the device is made by a 3D print device. Typical materials for 3D printing are silicones, polyurethanes and hydrogels. According to the invention, the individual device consists of elastomers, preferably of one and/or more crosslinking silicones and/or polyurethanes and/or hydrogels, also referred to as an emulsion with crosslinking agents as ink or 3D printing ink.

The device may be produced by additive manufacturing techniques. Additive manufacturing techniques comprise a process involving putting together or combining materials in order to manufacture 3D products from 3D modeling data, typically a computer-assisted design file, usually layer by layer.

In order to fit accurate to the individual subject's outer ear, ear scanning is an important step in manufacturing the device. The detailed geometry of the subject's ear needs to be obtained in order to acquire a perfect model of the individual's ear geometry. Thus, according to the invention, the individual's ear geometry encompasses the external ear, including the helix, the concha, the tragus, and the lobule and is obtained by scanning the external ear. The scan of the external ear is then converted into a model of the device e.g., to a computer-aided design CAD model or other digital representation of the model directly into the physical device. The model of the device may then be manufactured, preferably via additive manufacturing techniques, such as 3D printing.

Additive manufacturing systems make a three-dimensional 3D object through the solidification of layers of build material. Additive manufacturing systems make objects based on data in a 3D model of the object generated, for example, with a computer-aided drafting CAD computer program product. The model data is processed into slices, each slice defining portions of a layer of build material that is to be solidified.

The 3D-printing method may be a method step in the manufacturing of the device in one batch by additive manufacturing using a layer-by-layer technique and using a 3D printing device.

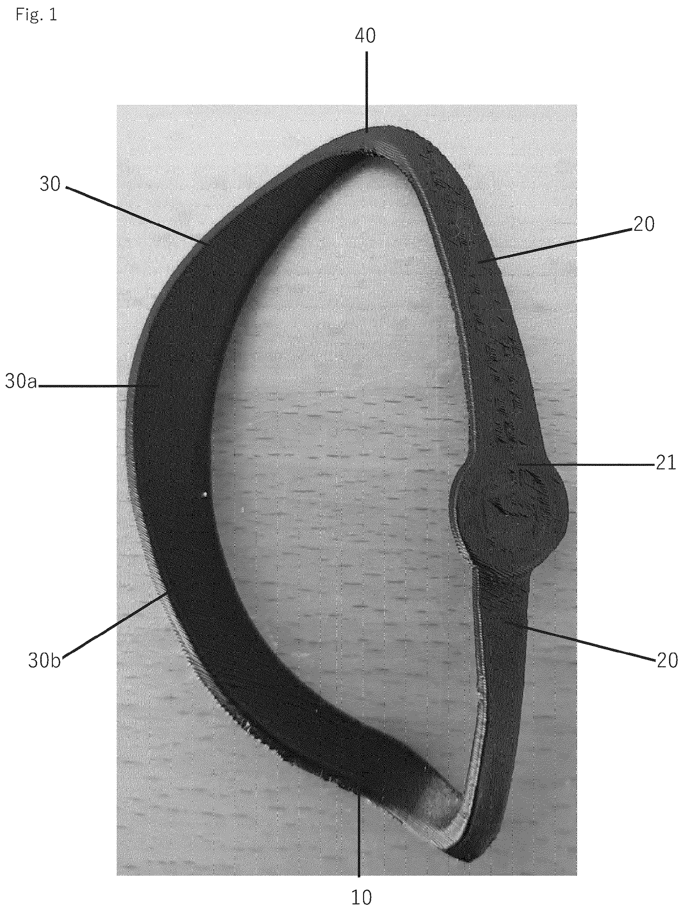

According to the invention, a composition of a silicone 3D printing ink is provided as an additive manufacturing ink, which is free of any solvents, biocompatible with the human body and non-toxic, used for the manufacture of such a device Fig. 1.

3D printed silicone removes the model and mold steps entirely, and simply prints the final part. Silicone materials for 3D printing must provide specific material properties, e.g., excellent thermal stability, available in different hardness's, water resistance and suitability as a sealant, UV resistance, oxidation resistance, compression and shear resistance, flame retardancy, electrical insulation, biocompatibility, optionally suited for sterilization, color and translucency options. The silicone material may be blended with additives such as curing agent or other polymers. One suitable material is for example, ACEO^{®} Silicones Wacker Chemie AG.

### Examples

The Examples which follow are set forth to aid in the understanding of the invention but are not intended to, and should not be construed to limit the scope of the invention in any way.

### Case Study

A longitudinal observation study is investigating the temporal development of tinnitus and related symptoms during a combination treatment. The combined treatment consists of the wearing the device around the ears during sleep together with a smartphone app that provides self-help tips to the participants. The participants provide self-reports about tinnitus loudness, tinnitus stress, mood, stress level, tensions in the jaw and neck muscles as outlined in Table 1. In addition to these questions, participants answer if they were using the device and entered comments in a free text field.

**Table 1. Questions for ecological momentary assessment**

| No | Question | Answer Options |
|---|---|---|
| 1 | How loud do you currently perceive the tinnitus? | Visual slider on a scale from 0 (low) to 10 (high) |
| 2 | How stressful is the tinnitus at the moment? | Visual slider on a scale from 0 (low) to 10 (high) |
| 3 | What is your current mood? | 5 smileys from very sad (rating 1) to very happy (rating 5) |
| 4 | How stressed do you feel right now? | Visual slider on a scale from 0 (no stress) to 10 (maximum stress level) |
| 5 | How tense does your jaw feel right now? | Visual slider on a scale from 0 (no tense at all) to 10 (very tensed) |
| 6 | How tense does your neck feel right now? | Visual slider on a scale from 0 (no tense at all) to 10 (very tensed) |

The treatment and app assessment are done under naturalistic conditions and the data collection is reduced to a minimum to assess the development of tinnitus and other health conditions with high ecological validity and low intrusion to everyday life. Only the short questions within the app needs to be answered by the participants.

## Claims

1. A method for producing an individual device (10) for improving health conditions in a subject, comprising:
scanning the geometry of the outer ears;
producing a 3D-model of the individual device (10); and
manufacturing the device (10);
wherein the device (10) is specifically adapted to the individual geometry of the outer ears of the subject;
to apply pressure to the external ear in a way that the position of the pinna is changed;
to exert pressure on acupressure points around to the external ear;
wherein the device (10) comprises an approximately straight portion (20) having circular portion (21), and a curved portion (30), and wherein the device is configured to surround the outer ear;
wherein the straight portion (20) is adapted to rest in front of the ear;
wherein the curved portion (30) is adapted to rest behind the ear;
wherein the circular portion (21) is located in the middle of the straight portion (20) and is adapted to exert pressure on the tragus; and wherein the device (10) is flexible.

2. The method according to claim 1, wherein the curved portion (30) has a smaller diameter at the beginnings and thickens towards the middle.

3. The method according to claim 1 or 2, wherein the device (10) is manufactured as one piece.

4. An individual device (10) for improving health conditions in a subject, wherein the device is produced by a method according to any one of claims 1 to 3, wherein the device (10) is adapted to fit accurately to the individual subject's outer ear; wherein the device (10) is specifically adapted to the individual geometry of the outer ears of the subject to apply pressure to the external ear in a way that the position of the pinna is changed, and to exert pressure on acupressure points around to the external ear; wherein the device (10) comprises a straight portion (20), which is adapted to rest in front of the ear;
a curved portion (30), which is adapted to rest behind the ear;
a circular portion (21), which is located in the middle of the straight portion (20) and is adapted to exert pressure on the tragus; and wherein the device (10) is flexible.

5. The individual device (10) of claim 4, wherein the approximately straight portion (20) of the device (10) is adapted to rest in front of the ear and the curved portion (30) is adapted to rest behind the ear.

6. The individual device of claim 4 or 5, wherein the curved portion (30) is adapted to stimulate various acupressure points behind the ear.

7. The individual device (10) of any one of claims 4 to 6, wherein the circular portion (21) is adapted to stimulate acupressure points in front ear.

8. The individual device (10) of any one of claims 4 to 6, wherein the circular portion (21) is adapted to stimulate the auricular vagus nerve.

9. The individual device (10) of any one of claims 4 to 8, wherein the device is adapted for use in improving health conditions in a subject.

10. The individual device (10) according to claim 9, wherein the curvec portion (30) of the device is adapted change the position of the pinna and/or stimulate various auricular acupressure points.

11. The individual device (10) according to claim 10, wherein the device is adapted for the treatment of tinnitus.

12. The individual device (10) according to claim 11, wherein the device is adapted to stimulate the vagus nerve for affecting multiple physiological functions.

13. The individual device (10) for use according to claim 12, wherein the multiple physiological functions are selected from the group consisting of neurological disorder, psychometric functions, metabolic syndrome, cardiovascular disorders, and pain.

## Patentansprüche

1. Verfahren zur Herstellung einer individuellen Vorrichtung (10) zur Verbesserung des Gesundheitszustandes in einem Subjekt, umfassend:
Abtasten der Geometrie der äußeren Ohren;
Erstellen eines 3D-Modells der individuellen Vorrichtung (10); und
Herstellen der Vorrichtung (10);
wobei die Vorrichtung (10) im Speziellen an die Geometrie der äußeren Ohren des Subjekts angepasst ist;
um Druck auf das äußere Ohr auf solche Weise auszuüben, dass die Position der Pinna verändert wird;
um Druck auf Akupressurpunkte um das äußere Ohr herum auszuüben;
wobei die Vorrichtung (10) einen ungefähr geraden Abschnitt (20), der einen kreisförmigen Abschnitt (21) aufweist, sowie einen gekrümmten Abschnitt (30) umfasst, und wobei die Vorrichtung dazu ausgestaltet ist, das äußere Ohr zu umgeben;
wobei der gerade Abschnitt (20) dazu angepasst ist, vor dem Ohr zu verbleiben;
wobei der gekrümmte Abschnitt (30) dazu angepasst ist, hinter dem Ohr zu verbleiben;
wobei sich der kreisförmige Abschnitt (21) in der Mitte des geraden Abschnitts (20) befindet und dazu angepasst ist, Druck auf den Tragus auszuüben;
und wobei die Vorrichtung (10) flexibel ist.

2. Verfahren nach Anspruch 1, wobei der gekrümmte Abschnitt (30) einen geringeren Durchmesser an den Anfängen aufweist und sich zu der Mitte hin verdickt.

3. Verfahren nach Anspruch 1 oder 2, wobei die Vorrichtung (10) in einem Stück hergestellt wird.

4. Individuelle Vorrichtung(10) zur Verbesserung des Gesundheitszustands in einem Subjekt, wobei die Vorrichtung gemäß einem Verfahren nach einem der Ansprüche 1 bis 3 hergestellt ist, wobei die Vorrichtung (10) dazu angepasst ist, exakt auf das äußere Ohr eines individuellen Subjekts zu passen; wobei die Vorrichtung (10) im Speziellen an die individuelle Geometrie der äußeren Ohren des Subjekts angepasst ist, wobei die Vorrichtung (10) im Speziellen an die individuelle Geometrie der äußeren Ohren des Subjekts angepasst ist, um Druck auf das äußere Ohr auf solche Weise auszuüben, dass die Position der Pinna verändert wird, und um Druck auf Akupressurpunkte um das äußere Ohr herum auszuüben; wobei die Vorrichtung (10) einen geraden Abschnitt (20) umfasst, der dazu angepasst ist, vor dem Ohr zu verbleiben;
einen gekrümmten Abschnitt (30), der dazu angepasst ist, hinter dem Ohr zu verbleiben;
einen kreisförmigen Abschnitt (21), der sich in der Mitte des geraden Abschnitts (20) befindet und dazu angepasst ist, Druck auf den Tragus auszuüben; und
wobei die Vorrichtung (10) flexibel ist.

5. Individuelle Vorrichtung (10) nach Anspruch 4, wobei der ungefähr gerade Abschnitt (20) der Vorrichtung (10) dazu angepasst ist, vor dem Ohr zu verbleiben, und der gekrümmte Abschnitt (30) dazu angepasst ist, hinter dem Ohr zu verbleiben.

6. Individuelle Vorrichtung nach Anspruch 4 oder 5, wobei der gekrümmte Abschnitt (30) dazu angepasst ist, verschiedene Akupressurpunkte hinter dem Ohr zu stimulieren.

7. Individuelle Vorrichtung (10) nach einem der Ansprüche 4 bis 6, wobei der kreisförmige Abschnitt (21) dazu angepasst ist, Akupressurpunkte vor dem Ohr zu stimulieren.

8. Individuelle Vorrichtung (10) nach einem der Ansprüche 4 bis 6, wobei der kreisförmige Abschnitt (21) dazu angepasst ist, den aurikulären Vagus-Nerv zu stimulieren.

9. Individuelle Vorrichtung (10) nach einem der Ansprüche 4 bis 8, wobei die Vorrichtung zur Verwendung bei der Verbesserung des Gesundheitszustands in einem Subjekt angepasst ist.

10. Individuelle Vorrichtung (10) nach Anspruch 9, wobei der gekrümmte Abschnitt (30) der Vorrichtung dazu angepasst ist, die Position der Pinna zu verändern und/oder verschiedene aurikuläre Akupressurpunkte zu stimulieren.

11. Individuelle Vorrichtung (10) nach Anspruch 10, wobei die Vorrichtung für die Behandlung von Tinnitus angepasst ist.

12. Individuelle Vorrichtung (10) nach Anspruch 11, wobei die Vorrichtung dazu angepasst ist, den Vagus-Nerv zu stimulieren, um mehrere physiologische Funktionen zu beeinflussen.

13. Individuelle Vorrichtung (10) zur Verwendung nach Anspruch 12, wobei die mehreren physiologischen Funktionen aus der Gruppe ausgewählt sind, die aus neurologischen Störungen, psychometrischen Funktionen, metabolischem Syndrom, kardiovaskulären Störungen und Schmerzen besteht.

## Revendications

1. Procédé de production d'un dispositif personnel (10) permettant d'améliorer des problèmes de santé chez un sujet, comprenant :
le scannage de la géométrie des oreilles externes ;
la production d'un modèle 3D du dispositif personnel (10) ; et
la fabrication du dispositif (10) ;
dans lequel le dispositif (10) est spécifiquement adapté à la géométrie individuelle des oreilles externes du sujet ;
pour appliquer une pression sur l'oreille externe de manière à ce que la position du pavillon soit changée ;
pour exercer une pression sur des points d'acupression autour de l'oreille externe ;
dans lequel le dispositif (10) comprend une partie approximativement droite (20) présentant une partie circulaire (21) et une partie incurvée (30), et dans lequel le dispositif est conçu pour entourer l'oreille externe ;
dans lequel la partie droite (20) est adaptée à rester devant l'oreille ;
dans lequel la partie incurvée (30) est adaptée à rester derrière l'oreille ;
dans lequel la partie circulaire (21) est située au milieu de la partie droite (20) et est adaptée à l'exercice d'une pression sur le tragus ;
et dans lequel le dispositif (10) est souple.

2. Procédé selon la revendication 1, dans lequel la partie incurvée (30) présente un diamètre plus petit au début et s'épaissit en direction du milieu.

3. Procédé selon la revendication 1 ou 2, dans lequel le dispositif (10) est fabriqué sous la forme d'une seule pièce.

4. Dispositif personnel (10) permettant d'améliorer des problèmes de santé chez un sujet, dans lequel le dispositif est produit par un procédé selon l'une quelconque des revendications 1 à 3, dans lequel le dispositif (10) est adapté à correspondre avec précision à l'oreille externe personnelle du sujet ; dans lequel le dispositif (10) est spécifiquement adapté à la géométrie personnelle des oreilles externes du sujet pour appliquer une pression sur l'oreille externe de manière à ce que la position du pavillon soit changée, et pour exercer une pression sur des points d'acupression autour de l'oreille externe ; dans lequel le dispositif (10) comprend une partie droite (20), qui est adaptée à rester devant l'oreille ;
une partie incurvée (30) qui est adaptée à rester derrière l'oreille ;
une partie circulaire (21), qui est située au milieu de la partie droite (20) et est adaptée à exercer une pression sur le tragus ; et
dans lequel le dispositif (10) est souple.

5. Dispositif individuel (10) selon la revendication 4, dans lequel la partie approximativement droite (20) du dispositif (10) est adaptée à rester devant l'oreille et la partie incurvée (30) est adaptée à rester derrière l'oreille.

6. Dispositif individuel selon la revendication 4 ou 5, dans lequel la partie incurvée (30) est adaptée à stimuler divers points d'acupression derrière l'oreille.

7. Dispositif individuel (10) selon l'une quelconque des revendications 4 à 6, dans lequel la partie circulaire (21) est adaptée à stimuler des points d'acupression devant l'oreille.

8. Dispositif individuel (10) selon l'une quelconque des revendications 4 à 6, dans lequel la partie circulaire (21) est adaptée à stimuler le nerf vague auriculaire.

9. Dispositif individuel (10) selon l'une quelconque des revendications 4 à 8, dans lequel le dispositif est adapté à être utilisé dans l'amélioration de problèmes de santé chez un sujet.

10. Dispositif individuel (10) selon la revendication 9, dans lequel la partie incurvée (30) du dispositif est adaptée à changer la position du pavillon et/ou stimuler divers points d'acupression auriculaires.

11. Dispositif individuel (10) selon la revendication 10, dans lequel le dispositif est adapté au traitement d'un acouphène.

12. Dispositif individuel (10) selon la revendication 11, dans lequel le dispositif est adapté à stimuler le nerf vague pour toucher de multiples fonctions physiologiques.

13. Dispositif individuel (10) selon la revendication 12, dans lequel les multiples fonctions physiologiques sont choisies dans le groupe constitué par un trouble neurologique, les fonctions psychométriques, le syndrome métabolique, les troubles cardiovasculaires et la douleur.
